# EUROPÄISCHE PATENTSCHRIFT

(11) **EP 2 065 363 B1**
(45) Veröffentlichungstag und Bekanntmachung des Hinweises auf die Patenterteilung: **22.07.2015**
(21) Anmeldenummer: 07023228.5
(22) Anmeldetag: 30.11.2007
(51) Int. Cl.: C07C 233/20, A61K 6/083, C09J 133/26

(54) **Dentalmaterialien auf der Basis von Alkylendiamin-N,N,N',N'-tetraessigsäure-(meth)acrylamiden**
Dental materials based on alkylenediamine-N,N,N',N'-tetraacetic acid-(meth)acrylamides
Matériaux dentaires à base d'alkylènediamine-N,N,N',N'-tétraacétique-(méth)acrylamides

(43) Veröffentlichungstag der Anmeldung: 03.06.2009
(73) Patentinhaber: Ivoclar Vivadent AG, 9494 Schaan (LI)
(72) Erfinder: Moszner, Norbert, Prof. Dr., 9495 Triesen (LI); Angermann, Jörg, Dr., 7320 Sargans (CH); Fischer, Urs Karl, 9320 Arbon (CH); Rheinberger, Volker, Dr., 9490 Vaduz (LI)
(74) Vertreter: UEXKÜLL & STOLBERG

(56) Entgegenhaltungen:
- EP-A- 1 374 828
- EP-A- 1 721 949
- US-A- 5 770 637
- Beyer/Walter: "Lehrbuch der Organischen Chemie", 1984, S, Hirzel Verlag, Stuttgard ISBN: 3-7776-0406-2 * Seite 243: Carbonsäureester, Darstellung *

## Beschreibung

Die vorliegende Erfindung betrifft Zusammensetzungen auf der Basis von Alkylendiamin-N,N,N',N'-tetraessigsäure-(meth)acrylamiden, die sich durch eine hohe Hydrolysestabilität auszeichnen und sich besonders als Dentalmaterialien eignen. Die Erfindung betrifft außerdem die Verwendung von Alkylendiamin-N,N,N',N'-tetraessigsäure-(meth)acrylamiden zur Herstellung von Dentalmaterialien.

Monofunktionelle Amide der Acryl- bzw. Methacrylsäure finden in unterschiedlichen Bereichen der Zahnmedizin Verwendung. So wurden verschiedene Acryl- oder Methacrylamide als Comonomere für die Herstellung von Polymeren für Prothesen vorgesehen (GB 1 039 750). Mehrfachfunktionelle (Meth)acrylamide, z.B. Bisacrylamide, sind als geeignete Vernetzerkomponente für dentale Adhäsive oder Komposite u.a. in US 2002/014138 A1 und WO 02/13768 A2 beschrieben. Weiterhin eignen sich geeignet funktionalis.ierte Acryl- bzw. Methacrylamide als wirksame Haftkomponenten oder Comonomere in Dentaladhäsiven, vor allem carboxylgruppenhaltige (Meth)acrylamide, wie z.B. N-Acryloylasparaginsäure (Y. Totti et al., Dental Materials 19 (2003) 253-258) oder N-Methacryloyl-4-aminovaleriansäure (N. Nishiyama et al., Biomaterials 25 (2004) 5441-5447), und auch hydroxylgruppenhaltige (Meth)acrylamide, wie z.B. N-(2-Hydroxyethyl)-N-methyl-acrylamid, dessen Verwendung in der EP 1 374 828 A1 beschrieben ist.

Aus der Komplexometrie ist die Chelatkomplexbildung der Ethylendiamintetraessigsäure (EDTA) und die große Affinität des Dinatriumsalzes der EDTA zu Calcium-Ionen bekannt (vgl. RÖMPP Lexikon Chemie, 10 Aufl., Georg Thieme Verlag, Stuttgart und New York, 1997, S. 2221). Adhäsive Zusammensetzungen auf der Basis von (Meth)acrylsäureester-Derivaten von EDTA sind in DE 10 2005 022 172 A1 beschrieben, jedoch zeichnen sich diese Haftvermittlerkomponenten durch eine schlechte Hydrolysebeständigkeit aus.

Der Erfindung liegt die Aufgabe zugrunde, Dentalmaterialien bereitzustellen, die polymerisationsfähig sind, sich durch eine gute Löslichkeit auszeichnen und in Gegenwart von Wasser eine verbesserte Lagerstabilität zeigen. Zudem sollen die Materialien eine gute Haftung auf Dentin und Zahnschmelz zeigen.

Die Aufgabe wird erfindungsgemäß gelöst durch eine polymerisierbare Zusammensetzung, die mindestens ein Alkylendiamin-N,N,N',N'-tetraessigsäure-(meth)acrylamid mit der Formel (I) 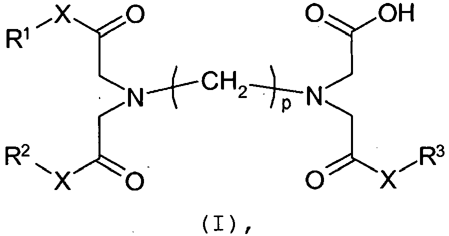 in der
X jeweils unabhängig für O oder vorzugsweise NR⁶ steht,
R¹, R² und R³ unabhängig voneinander für Wasserstoff oder 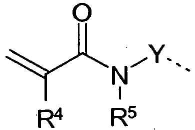 stehen, wobei R¹, R² und R³ nicht gleichzeitig Wasserstoff sind,
Y jeweils unabhängig für einen linearen oder verzweigten C₁-bis C₁₅-Alkylenrest steht, der durch ein oder mehrere O oder S unterbrochen sein kann,
R⁴ jeweils unabhängig für Wasserstoff oder einen C₁- bis C₁₀-Alkylrest steht,
R⁵ jeweils unabhängig für Wasserstoff oder einen C₁- bis C₈-Alkylrest steht,
R⁶ jeweils unabhängig für Wasserstoff oder einen C₁- bis C₆-Alkylrest steht und
p 2 bis 10 ist,
   mindestens ein Monomer mit 2 oder mehr polymerisierbaren Gruppen und/oder mindestens ein Monomer mit einer oder mehreren aciden Gruppen und
einen Initiator für die radikalische Polymerisation enthält.

Der Hinweis, dass ein Rest durch ein Heteroatom wie O oder S unterbrochen sein kann, ist so zu verstehen, dass die Heteroatome in die Kohlenstoffkette des Restes eingeschoben werden, d.h. beidseitig von Kohlenstoffatomen begrenzt werden. Die Anzahl der Heteroatome ist daher um mindestens 1 kleiner als die Zahl der Kohlenstoffatome, und die Heteroatome können nicht endständig sein.

Eine bevorzugte Ausführungsform der polymerisierbaren Zusammensetzung ist dadurch gekennzeichnet, dass
- Y: jeweils unabhängig für einen linearen oder verzweigten C₁-bis C₁₀-Alkylenrest steht, der durch ein oder mehrere O oder S unterbrochen sein kann,
- R⁴: jeweils unabhängig für Wasserstoff oder einen C₁- bis C₈-Alkylrest steht,
- R⁵: jeweils unabhängig für Wasserstoff oder einen C₁- bis C₆-Alkylrest steht,
- R⁶: jeweils unabhängig für Wasserstoff oder einen C₁- bis C₄-Alkylrest steht und
- p: 2 bis 6 ist.

Besonders bevorzugt steht Y für einen linearen oder verzweigten C₁- bis C₆-Alkylenrest, insbesondere einen linearen oder verzweigten C₂- bis C₅-Alkylenrest, am meisten bevorzugt einen Ethylenrest.

Besonders bevorzugt steht R⁴ für Wasserstoff oder einen C₁- bis C₄-Alkylrest, insbesondere für Wasserstoff oder einen Methylrest.

Besonders bevorzugt steht R⁵ für Wasserstoff oder einen C₁- bis C₄-Alkylrest, insbesondere für Wasserstoff oder einen Methylrest.

Besonders bevorzugt steht R⁶ für Wasserstoff oder einen C₁- bis C₃-Alkylrest, insbesondere für Wasserstoff oder einen Methylrest.

Besonders bevorzugt ist p 2 bis 4, am meisten bevorzugt 2.

Bei den genannten Alkyl- und Alkylenresten handelt es sich vorzugsweise um lineare Gruppen.

Es ist besonders bevorzugt, dass R¹ für Wasserstoff steht und R² und R³ jeweils für 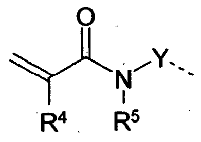 stehen. Es ist weiter bevorzugt, dass R¹ und das diesem benachbarte X zusammen für OH stehen. Noch weiter bevorzugt stehen die übrigen X jeweils unabhängig für NR⁶.

Alkylendiamin-N,N,N',N'-tetraessigsäure-(meth)acrylamide mit der Formel (I) lassen sich analog zu grundsätzlich bekannten Syntheseverfahren herstellen.

So erfolgt zunächst ausgehend von der Alkylendiamin-N,N,N',N'-tetraessigsäure die Herstellung des entsprechenden bicyclischen Anhydrids durch Umsetzung mit Acetanhydrid (Ac₂O), z.B. analog der CS 272 584 B1:

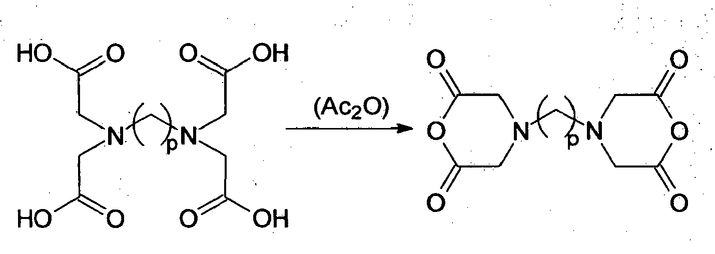

### Konkretes Beispiel:

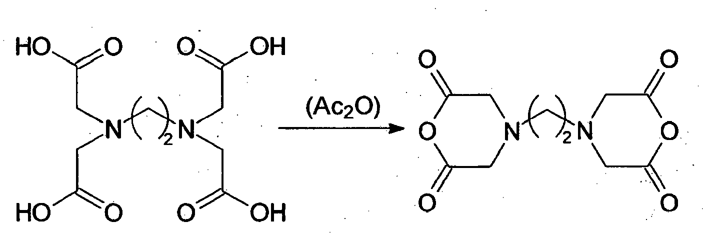

Das bicyclische Anhydrid der Alkylendiamin-N,N,N',N'-tetraessigsäure wird dann in einer zweiten Reaktionsstufe mit einem oder mehreren geeigneten XH-funktionalisierten. (X = O, NR⁶) (Meth)acrylamiden zu einem entsprechenden Alkylendiamin-N,N,N',N'-tetraessigsäure-(meth)acrylamid mit der Formel (I) umgesetzt:

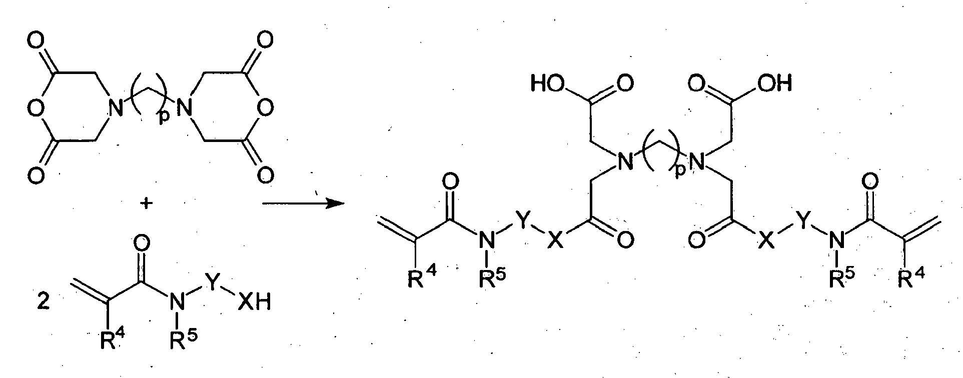

Die in der 2. Reaktionsstufe eingesetzten XH-Gruppen-haltigen (Meth)acrylamid-Derivate (X = O, NR⁶) lassen sich in einfacher Weise aus den entsprechenden Aminoalkanolen (X = O) bzw. linearen oder verzweigten aliphatischen Diaminen (X = NH oder NR) unter Anwendung der aus der organischen Chemie bekannten Methoden für die Knüpfung von Amid-Bindungen (vgl. Methoden der Organischen Chemie, HOUBEN-WEYL Bd. E5 1985, Georg Thieme Verlag S. 941 ff.) durch Umsetzung mit (Meth)acrylsäurechlorid bzw. -anhydrid herstellen.

### Konkretes Beispiel:

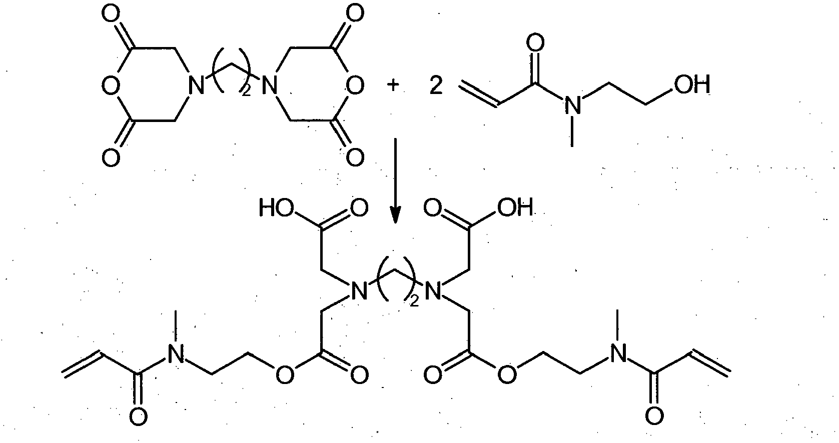

Beispiele für die erfindungsgemäß verwendeten Alkylendiamin-N,N,N',N'-tetraessigsäure-(meth)acrylamide mit der Formel (I) sind u.a.:

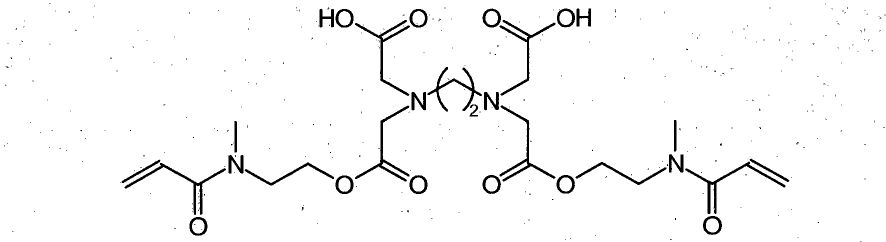

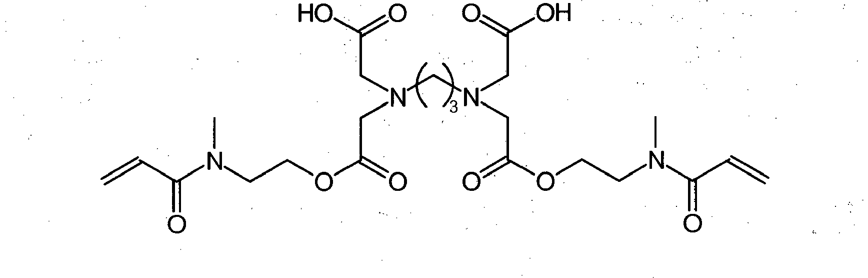

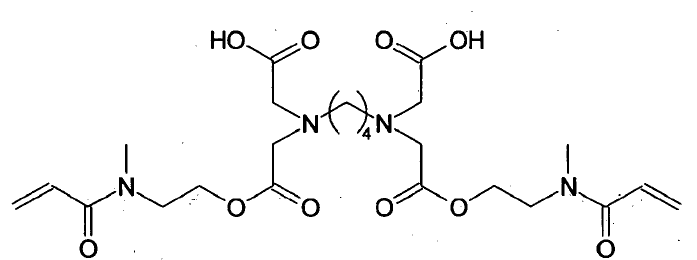

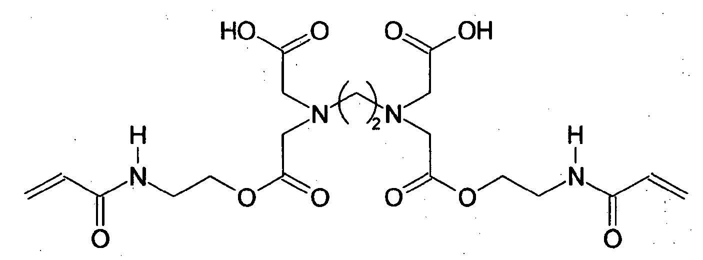

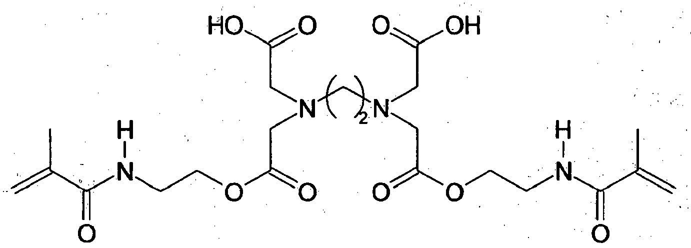

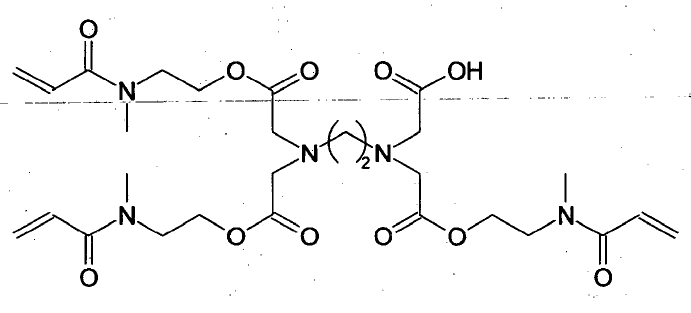

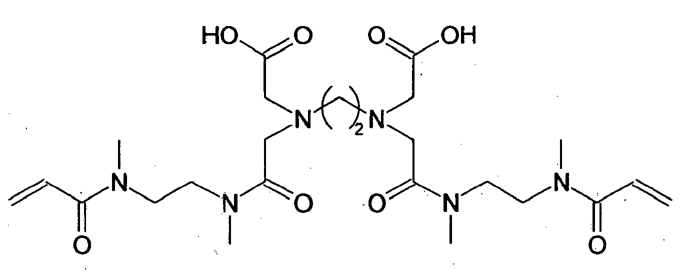

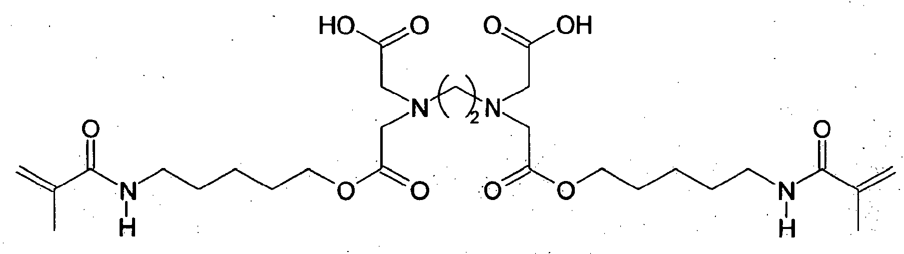

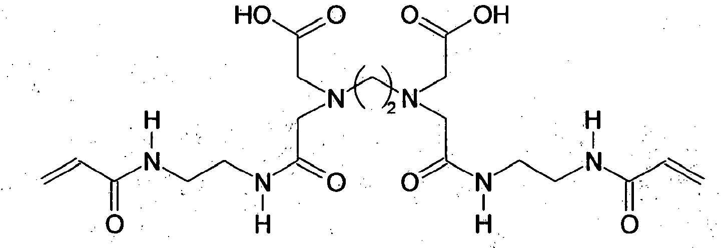

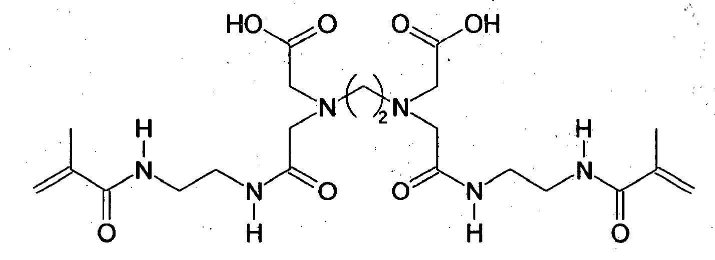

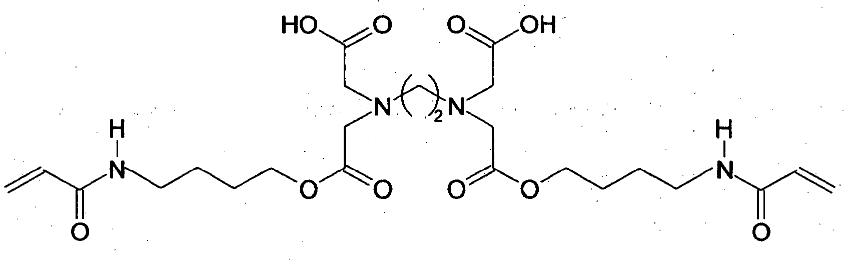

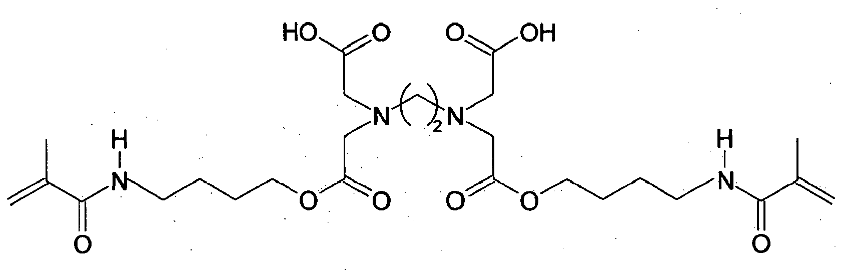

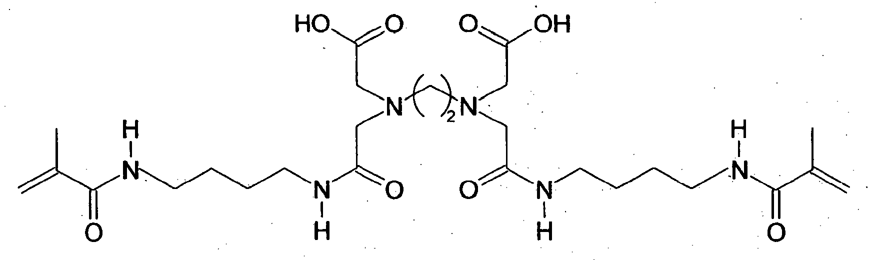

Die Alkylendiamin-N,N,N',N'-tetraessigsäure-(meth)acrylamide sind sehr gut in Wasser oder Mischungen von Wasser mit polaren Lösungsmitteln, wie Aceton, Ethanol, Acetonitril oder Tetrahydrofuran (THF) löslich. Sie weisen insbesondere eine hohe Affinität zu Calciumionen auf, die sie unter Bildung entsprechender Salze komplexieren können. Aufgrund ihrer hohen Affinität zu Calciumionen sind die Alkylendiamin-N,N,N',N'-tetraessigsäure-(meth)acrylamide in der Lage, Haftung auf Zahnschmelz und Dentin zu vermitteln.

In den Monomeren der Formel (I) ist mindestens eine saure COOH-Gruppe kovalent mit mindestens einer polymerisationsfähigen (Meth)acrylamidgruppe verbunden, die unter wässrigen Bedingungen bei Raumtemperatur eine hohe Hydrolysestabilität aufweist. Aufgrund der Hydrolysestabilität der Alkylendiamin-N,N,N',N'-tetraessigsäure-(meth)acrylamide lassen sich daraus bei Raumtemperatur lagerstabile Mischungen mit Wasser und anderen hydrolysestabilen Komponenten herstellen, die als Adhäsive, Zemente, Beschichtungsmaterialien oder Komposite vor allem im Dentalbereich verwendet werden können.

Die erfindungsgemäßen Zusammensetzungen enthalten vorzugsweise 0,5 bis 50 Gew.-%, vorzugsweise 5 bis 40 Gew.-% und ganz besonders bevorzugt 5 bis 30 Gew.-% Alkylendiamin-N,N,N',N'-tetraessigsäure-(meth)acrylamid gemäß Formel (I).

Neben dem Alkylendiamin-N,N,N',N'-tetraessigsäure-(meth)acrylamid gemäß Formel (I) enthalten die erfindungsgemäßen Zusammensetzungen vorzugsweise mindestens ein weiteres polymerisierbares Monomer. Als weitere polymerisierbare Monomere eignen sich besonders radikalisch polymerisierbare Monomere.

Diese radikalisch polymerisierbaren Monomere können eine oder mehrere radikalisch polymerisierbare Gruppen aufweisen, wobei Zusammensetzungen, die mindestens ein Monomer mit 2 oder mehr, vorzugsweise 2 bis 5 radikalisch polymerisierbaren Gruppen enthalten, bevorzugt sind. Monomere mit 2 oder mehr polymerisierbaren Gruppen wirken als Vernetzer und erhöhen so die mechanische Stabilität der gehärteten Zusammensetzungen.

Bevorzugte Vernetzermonomere sind Bis-GMA, Glycerindimethacrylat, und besonders Urethane aus 2-(Hydroxymethyl)acrylsäure und Diisocyanaten, wie 2,2,4-Trimethyl-hexamethy-lendiisocyanat oder Isophorondiisocyanat, vernetzende Pyrrolidone, wie z.B. 1,6-Bis(3-vinyl-2-pyrrolidonyl)-hexan, oder kommerziell zugängliche Bis(meth)acrylamide, wie Methylen- oder Ethylen bisacrylamid, N,N'-Diethyl-1,3-bis(acrylamido)-propan, 1,3-Bis(methacrylamido)-propan, 1,4-Bis(acrylamido)-butan, 1,4-Bis-(acryloyl)-piperazin, 2,6-Dimethylen-4-oxa-heptan-1,7-dicarbonsäure-bis-(propylamid), 1,6-Bis-(acrylamido)-2,2,4(2,4,4)-trimethylhexan und N,N'-Dimethyl-1,6-bis-(acrylamido)-hexan.

Zusammensetzungen, die neben dem Alkylendiamin-N,N,N',N'-tetraessigsäure-(meth)acrylamid gemäß Formel (I) 1 bis 45 Gew.-%, vorzugsweise 5 bis 30 Gew.-% und ganz besonders bevorzugt zugt 5 bis 20 Gew.-% Vernetzermonomer enthalten, insbesondere Bis(meth)acrylamid, sind erfindungsgemäß besonders bevorzugt. Diese und, wenn nicht anders angegeben, alle anderen Prozentangaben beziehen sich auf die Gesamtmasse der Zusammensetzung.

Als radikalisch polymerisierbare Monomere sind weiterhin bei Raumtemperatur flüssige Monomere bevorzugt, die sich als Verdünnermonomere eignen. Bevorzugt sind Monomere mit einer Viskosität von 0,01 bis 10 Pa·s bei Raumtemperatur, insbesondere mono- oder mehrfach funktionelle (Meth)acrylate. Besonders bevorzugt sind Mono(meth)acrylate und auf Grund ihrer Hydrolysebeständigkeit insbesondere Mesitylmethacrylat und 2-(Alkoyxymethyl)acrylsäuren, z.B. 2-(Ethoxymethyl)acrylsäure, 2-(Hydroxymethyl)acrylsäure und Allylether. Weitere bevorzugte hydrolysestabile Monomere sind N-Vinylpyrrolidon, N-monoalkylsubstituierte (Meth)acrylamide, wie z.B. N-Ethylacrylamid, N-(2-Hydroxyethyl)acrylamid, N-Ethylmethacrylamid oder N-(2-Hydroxyethyl)methacrylamid, und N,N-dialkylsubstituierte Acrylamide, wie z.B. N-Methyl-N-(2-hydroxyethyl)acrylamid oder das kommerziell zugängliche, dünnflüssige N,N-Dimethylacrylamid. Unter Alkyl werden bevorzugt Reste mit 1 bis 6, insbesondere 1 bis 3 Kohlenstoffatomen verstanden.

Die erfindungsgemäßen Zusammensetzungen enthalten neben dem Alkylendiamin-N,N,N',N'-tetraessigsäure-(meth)acrylamid gemäß Formel (I) vorzugsweise 0 bis 50 Gew.-%, vorzugsweise 10 bis 40 Gew.-% und ganz besonders bevorzugt 10 bis 30 Gew.-% Verdünnungsmonomer.

Gemäß einer weiteren bevorzugten Ausführungsform enthalten die Zusammensetzungen mindestens ein acides radikalisch polymerisierbares Monomer, d.h. ein Monomer mit einer oder mehreren aciden Gruppen, wie Carbonsäureanhydrid-, Carbonsäure-, Phosphorsäure-, Dihydrogenphosphat-, Phosphonsäure- und Sulfonsäuregruppen. Bevorzugte acide Gruppen sind Carbonsäure-, Phos-phorsäure- und Phosphonsäuregruppen. Besonders bevorzugte acide Monomere sind 4-(Meth)acryloyloxyethyltrimellitsäureanhydrid, 10-Methacryloyloxydecylmalonsäure, N-(2-Hydroxy-3-methacryloyloxypropyl)-N-phenylglycin, 4-Vinylbenzoesäure, 2-Methacryloyloxyethyl-phenyl-hydrogenphosphat, 10-Methacryloyloxydecyl-dihydrogenphosphat oder Dipentaerythritol-pentamethacryloyloxyphosphat (PENTA), Vinylsulfonsäure und 4-Vinylbenzolsulfonsäure. Auf Grund ihrer Hydrolysestabilität sind 4-Vinylbenzylphosphonsäure und 2-[4-(Dihydroxyphosphoryl)-2-oxabutyl]-acrylsäure (DHPOBAE) sowie deren Amide und hydrolysestabile Ester, wie z.B. 2-[4-(Dihydroxyphosphoryl)-2-oxa-butyl]-acrylsäure-2,4,6-trimethylphenylester, sowie (Meth)acrylamid-dihydrogenphosphate, wie z.B. 6-Methacrylamidohexanyl- oder 1,3-Bis(methacrylamido)-propan-2-yl-dihydrogenphosphat, besonders bevorzugt.

Zusammensetzungen, die neben dem Alkylendiamin-N,N,N',N'-tetraessigsäure-.(meth)acrylamid gemäß Formel (I) 1 bis 50 Gew.-%, vorzugsweise 5 bis 40 Gew.-% und ganz besonders bevorzugt 10 bis 30 Gew.-% acides Monomer enthalten, insbesondere acides Monomer mit Dihydrogenphosphat-, Phosphonsäure- und/oder Sulfonsäuregruppen, sind erfindungsgemäß besonders bevorzugt.

Die genannten Monomere können jeweils allein oder als Mischung eingesetzt werden. Erfindungsgemäß sind besonders solche Monomere bevorzugt, die eine hohe Hydrolysestabilität aufweisen. Im Zusammenhang mit der vorliegenden Erfindung werden solche Verbindungen als hydrolysestabil bezeichnet, die in Wasser oder in Mischungen von Wasser und wassermischbaren Lösungsmitteln bei einer Konzentration von ca. 20 Masse-% bei 37 °C für mindestens 8 Wochen stabil sind. Diese Hydrolysestabilität zeigen vor allem polymerisierbare Carbonsäureamide und Mesitylester, deren Esterhydrolyse sterisch gehemmt ist.

Zur Initiierung der Polymerisation enthalten die erfindungsgemäßen Zusammensetzungen einen Initiator für die radikalische Polymerisation, besonders bevorzugt einen Initiator für die Photopolymerisation. Hierzu eignen sich insbesondere Benzophenon, Benzoin sowie deren Derivate oder α-Diketone oder deren Derivate, wie 9,10-Phenanthrenchinon, 1-Phenyl-propan-1,2-dion, Diacetyl oder 4,4-Dichlorbenzil. Besonders bevorzugt werden Campherchinon und 2,2-Dimethoxy-2-phenyl-acetophenon und ganz besonders bevorzugt α-Diketone in Kombination mit Aminen als Reduktionsmittel eingesetzt. Bevorzugte Amine sind 4-(Dimethylamino)-benzoesäureester, N,N-Dimethylaminoethylmethacrylat, N,N-Dimethyl-sym.-xylidin und Triethanolamin.

Als Initiatoren für die Heisshärtung eignen sich auch Benzpinakol und 2,2'-Dialkylbenzpinakole. Als Initiatoren für eine bei Raum-temperatur durchgeführte Polymerisation werden Redox-Initiatorkombinationen, wie z.B. Kombinationen von Benzoylperoxid mit N,N-Dimethyl-sym.-xylidin oder N,N-Dimethyl-p-toluidin, verwendet. Darüber hinaus sind auch Redoxsysteme bestehend aus Peroxiden und solchen Reduktionsmitteln, wie z.B. Ascorbinsäure, Barbiturate oder Sulfinsäuren, besonders geeignet.

Zusammensetzungen, die neben dem Alkylendiamin-N,N,N',N'-tetraessigsäure-(meth)acrylamid gemäß-Formel (I) 0,01 bis 15 Gew.-%, vorzugsweise 0,1 bis 5,0 Gew.-% und ganz besonders bevorzugt 0,3 bis 3,0 Gew.-% Initiator für die radikalische Polymerisation enthalten sind erfindungsgemäß besonders bevorzugt.

Weiterhin können die erfindungsgemäßen Zusammensetzungen zur Verbesserung der mechanischen Eigenschaften oder zur Einstellung der Viskosität Füllstoff enthalten. Als Füllstoffe eignen sich organische und anorganische Partikel und Fasern.

Bevorzugt werden als Füllstoff partikuläre Materialien mit einer mittleren Partikelgröße von 1 nm bis 10 µm, vorzugsweise von 5 nm bis 5 µm verwendet. Der Begriff mittlere Partikelgröße bezieht sich hier auf das Volumenmittel.

Bevorzugte anorganische partikuläre Füllstoffe sind amorphe kugelförmige Materialien auf der Basis von Metalloxiden, wie ZrO₂ und TiO₂ bzw. Mischoxiden aus SiO₂, ZrO₂ und/oder TiO₂; mono-. disperse, nanopartikuläre Füllstoffe, vorzugsweise auf Basis von SiO₂, ZrO₂, Al₂O₃, AlO(OH) oder deren Mischoxiden, wie pyrogene Kieselsäure oder Fällungskieselsäure, vorzugsweise mit einer mittleren Partikelgröße von 5 bis 200 nm, besonders bevorzugt 10 bis 100 nm, ganz besonders bevorzugt 10 bis 50 nm; mikrofeine Füllstoffe, vorzugsweise auf Basis von Quarz-, Keramik-, Glaskeramik- oder Glaspulver, vorzugsweise mit einer mittleren Partikelgröße von 0,01 bis 5 µm, insbesondere 0,3 bis 5 µm, besonders bevorzugt 0,4 bis 3 µm und ganz besonders bevorzugt 0,4 bis 1 µm; sowie röntgenopake Füllstoffe, wie Ytterbiumtrifluorid oder nanopartikuläres Tantal(V)-oxid bzw. Bariumsulfat.

Die erfindungsgemäßen Zusammensetzungen können zudem Lösungsmittel, wie Wasser, Ethylacetat oder Ethanol, oder Lösungsmittelgemische enthalten. Dabei sind hydrolysestabile Lösungsmittel, wie Wasser oder Ethanol, oder Lösungsmittelgemische bevorzugt.

Darüber hinaus können die erfindungsgemäßen Zusammensetzungen weitere Additive enthalten, insbesondere Stabilisatoren, Aromastoffe, Farbmittel, mikrobiocide Wirkstoffe, fluoridionenabgebende Additive, optische Aufheller, Weichmacher und UV-Absorber.

Erfindungsgemäß sind solche Zusammensetzungen bevorzugt, die die folgenden Komponenten enthalten:
a) 0,5 bis 50 Gew.-%, bevorzugt 5 bis 40 Gew.-% und besonders bevorzugt 5 bis 30 Gew.-% Alkylendiamin-N,N,N',N'-tetraessig-, säure-(meth)acrylamid gemäß Formel (I),
b) 0,01 bis 15 Gew.-%, bevorzugt 0,1 bis 5,0 Gew.-%, besonders bevorzugt 0,3 bis 3,0 Gew.-% Initiator für die radikalische Polymerisation,
c) bis zu 80 Gew.-%, bevorzugt bis zu 60 Gew.-% und besonders bevorzugt 10 bis 50 Gew.-% weitere polymerisierbare Monomere,
d) 0 bis 95 Gew.-%, bevorzugt 0 bis 80 Gew.-% und besonders bevorzugt 10 bis 60 Gew.-% Lösungsmittel, vorzugsweise Wasser,
e) 0 bis 75 Gew.-% Füllstoff.

Die erfindungsgemäßen Zusammensetzungen eignen sich besonders als Dentalmaterialien, insbesondere als Adhäsive und Zemente, wie z.B. Befestigungszemente. Solche Adhäsive zeichnen sich durch eine sehr gute Haftung auf der Zahnhartsubstanz, d.h. auf Schmelz und Dentin, aus und sind in Gegenwart von Wasser oder Alkohol hydrolyse- und alkoholysestabil.

Die bevorzugte Zusammensetzung der Materialien für dentale Anwendungen richtet sich nach dem gewünschten Verwendungszweck.

Bevorzugte Adhäsive enthalten die folgenden Komponenten:
a) 0,5 bis 50 Gew.-%, bevorzugt 5 bis 40 Gew.-% und besonders bevorzugt 5 bis 30 Gew.-% Alkylendiamin-N,N,N',N'-tetraessigsäure-(meth)acrylamid gemäß Formel (I),
b) 0,01 bis 15 Gew.-%, bevorzugt 0,1 bis 5,0 Gew.-%, besonders bevorzugt 0,3 bis 3,0 Gew.-% Initiator für die radikalische Polymerisation,
c) bis zu 80 Gew.-%, bevorzugt bis zu 60 Gew.-% und besonders bevorzugt 10 bis 50 Gew.-% weitere polymerisierbare Monomere,
d) 0 bis 95 Gew.-%, bevorzugt 0 bis 80 Gew.-% und besonders bevorzugt 10 bis 60 Gew.-% Lösungsmittel, vorzugsweise Wasser,
e) 0 bis 60 Gew.-%, bevorzugt 0 bis 50 Gew.-% und besonders bevorzugt 0 bis 20 Gew.-% Füllstoff, vorzugsweise monodispersen, nanopartikulären Füllstoff auf Basis von SiO₂, ZrO₂, Al₂O₃, AlO(OH) oder deren Mischoxiden, vorzugsweise mit einer mittleren Partikelgröße von 5 bis 200 nm, besonders bevorzugt 10 bis 100 nm, ganz besonders bevorzugt 10 bis 50 nm.

Bevorzugte Zemente enthalten die folgenden Komponenten:
a) 0,5 bis 50 Gew.-%, bevorzugt 5 bis 40 Gew.-% und besonders bevorzugt 5 bis 30 Gew.-% Alkylendiamin-N,N,N',N'-tetraessigsäure-(meth)acrylamid gemäß Formel (I),
b) 0,01 bis 15 Gew.-%, bevorzugt 0,1 bis 5,0 Gew.-%, besonders bevorzugt 0,3 bis 3,0 Gew.-% Initiator für die radikalische Polymerisation,
c) bis zu 80 Gew.-%, bevorzugt bis zu 60 Gew.-% und besonders bevorzugt 10 bis 50 Gew.-% weitere polymerisierbare Monomere,
d) 1 bis 75 Gew.-%, bevorzugt 20 bis 75 Gew.-% und besonders bevorzugt 40 bis 65 Gew.-% Füllstoff, vorzugsweise mikrofeinen Füllstoff auf Basis von Quarz-, Keramik-, Glaskeramik- oder Glaspulver, vorzugsweise mit einer mittleren Teilchengröße von 0,01 bis 5 µm, insbesondere 0,3 bis 5 µm, besonders bevorzugt 0,4 bis 3 µm und ganz besonders bevorzugt 0,4 bis 1 µm.

Alle Prozentangaben beziehen sich auf die Gesamtmasse der Zusammensetzung. Als weitere polymerisierbare Monomere werden vorzugsweise eines oder mehrere der oben definierten Monomere eingesetzt, besonders bevorzugt in den dort jeweils definierten Mengen, wobei die Mengen der einzelnen Monomere so gewählt werden, dass die Gesamtmenge an zusätzlichem Monomer die oben definierten Bereiche nicht überschreitet. Ganz besonders bevorzugt sind Zusammensetzungen, die mindestens ein acides Monomer oder mindestens ein Vernetzermonomer, insbesondere mindestens ein acides Monomer und mindestens ein Vernetzermonomer oder mindestens ein acides Vernetzermonomer enthalten.

Die bevorzugten erfindungsgemäßen Zusammensetzungen härten unter Ausbildung von stark vernetzten Polymernetzwerken aus, die in Wasser wenig oder gar nicht quellen.

Die Erfindung betrifft auch die Verwendung eines Alkylendiamin-N,N,N',N'-tetraessigsäure-(meth)acrylamids mit der Formel (I) zur Herstellung eines Dentalmaterials, insbesondere zur Herstellung eines Adhäsivs oder Zements.

Die Erfindung wird im Folgenden anhand von Beispielen näher erläutert.

### Beispiele:

### Beispiel 1:

### Herstellung des EDTA-di(5-methacrylamidopentyl)esters MAM-EDTA

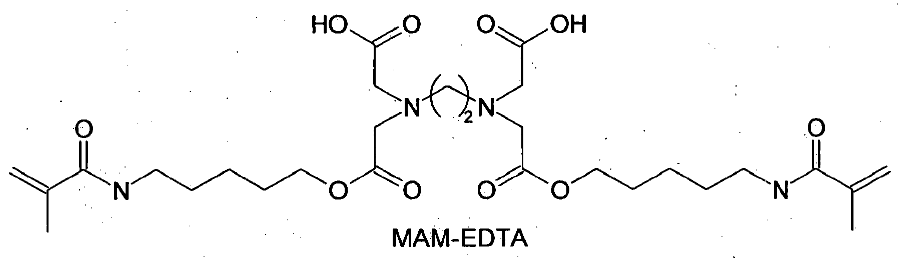

EDTA-Dianhydrid wurde gemäß dem Patent CS 272 584 B1 durch Umsetzung von EDTA mit Essigsäureanhydrid hergestellt. N-(5-Hydroxypentyl)methacrylamid wurde durch Umsetzung von kommerziell verfügbarem 5-Aminopentanol mit Methacrylsäureanhydrid erhalten.

Zu einer Lösung von 17,0 g (66,0 mmol) EDTA-Dianhydrid, 25,4 g (148 mmol) N-(5-Hydroxypentyl)methacrylamid und 50 mg BHT in 100 ml wasserfreiem Methylenchlorid wurden 3 ml Triethylamin getropft und das Gemisch anschließend bei Raumtemperatur 18 h gerührt. Die nach der Entfernung des Lösungsmittels (40 °C, 500-12 mbar) erhaltene braune, hochviskose Flüssigkeit wurde in 100 ml gesättigter Natriumchloridlösung aufgenommen und dreimal mit je 100 ml Tetrahydrofuran extrahiert. Nach dem Trocknen mit wasserfreiem Natriumsulfat und der Entfernung des Lösungsmittels (40°C, 300-12 mbar, anschließend Feinvakuum) wurden 27,5 g (70 % Ausbeute) an MAM-EDTA als klares, gelbes Harz erhalten.

¹H-NMR (400 MHz, DMSO-d₆): δ = 1,23-1,35, 1,39-1,50, 1,52-1,65 [3 m, je 4H, (CH₂)₃CH₂N], 1,84 (s, 6H, CH₃) , 2,78 [s, 4H, (CH₂)₂N], 3,07-3,12 [m, 4H, (CH₂)₃CH₂N], 3,47, 3,58 (2 s, je 4H, NCH₂C=O) , 4,01-4,04 (t, J = 6,6 Hz, 4H, OCH₂), 5, 29, 5, 62 (2 s, je 2H, =CH₂), 7,89 (t, J = 5,5 Hz, 2H, NH), 12,1 (br. s, 2H, OH).

### Vergleichsbeispiel 2:

### Herstellung des EDTA-di(2-methacryloyloxyethyl)esters MA-EDTA

(Referenzsubstanz)

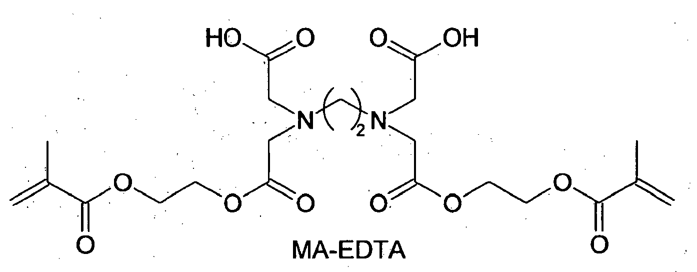

Die Synthese von MA-EDTA erfolgte analog der in der Offenlegungsschrift DE 10 2005 022 172 A1 angegebenen Vorschrift durch Umsetzung von EDTA-Dianhydrid mit 2-Hydroxyethylmethacrylat (HEMA). Dabei wurde MA-EDTA als weißer Feststoff (Schmp. 74-76°C) mit einer Ausbeute von 82 % erhalten.

¹H-NMR (400 MHz, DMSO-d₆): δ = 1,87 (s, 6H, CH₃) , 2,51 [s, 4H, (CH₂)₂N], 3,44, 3,58 (2 s, je 4H, NCH₂C=O) , 4,30 (s, 8H, OCH₂), 5,68, 6,04 (2 s, je 2H, =CH₂), 11, 1 (br. s, 2H, OH).

### Beispiel 3:

### Untersuchung der Hydrolysestabilität von MAM-EDTA aus Beispiel 1 und der Referenzsubstanz MA-EDTA aus Beispiel 2

Zur Untersuchung der Hydrolysestabilitäten der Monomeren MAM-EDTA bzw. MA-EDTA wurde je eine 20 %ige Lösung in DMSO-d₆/D₂O (1:1) bei 37°C gelagert. In bestimmten Zeitabständen wurden die ¹H-NMR-Spektren dieser Lösungen aufgenommen. Dabei zeigte sich, dass bei der HEMA-substituierten Referenzsubstanz MA-EDTA im ¹H-NMR-Spektrum sofort ein zweites Doppelbindungssignal zu beobachten war, welches durch die Hydrolyse der Methacrylestergruppe unter Abspaltung von Methacrylsäure hervorgerufen wird. Nach 119 Tagen Lagerung bei 37°C waren ca. 50 % der Methacrylestergruppen hydrolysiert. Demgegenüber war das erfindungsgemäße Monomer MAM-EDTA deutlich hydrolysestabiler. Zu Untersuchungsbeginn waren im ¹H-NMR-Spektrum von MAM-EDTA keine Hydrolyseprodukte erkennbar. Auch nach 119 Tagen bei 37°C konnten im ¹H-NMR-Spektrum keine zusätzlichen Doppelbindungssignale detektiert werden.

### Beispiel 4:

Herstellung eines Dentinadhäsivs auf der Basis von MAM-EDTA bzw. der Referenzsubstanz MA-EDTA

Zur Untersuchung der Dentinhaftung auf Rinderzahndentin wurden zwei Adhäsive folgender Zusammensetzung (Angabe in Masse-%) hergestellt, die entweder MAM-EDTA oder MA-EDTA (Vergleichsbeispiel) enthalten:

| Komponente | erfindungsgemäßes Beispiel | Vergleichs beispiel |
|---|---|---|
| MAM-EDTA | 30,7 % | - |
| MA-EDTA | - | 30,7 % |
| HEMA | 14,8 % | 14,8 % |
| UDMA¹⁾ | 7,4 % | 7,4 % |
| Bis-GMA²⁾ | 7,4 % | 7,4 % |
| Aceton | 29,6 % | 29,6 % |
| Wasser | 7,4 % | 7,4 % |
| Photoinitiator ³⁾ | 2,7 % | 2,7 % |

| | | |
|---|---|---|
| ¹⁾ UDMA - Umsetzungsprodukt von 2 mol HEMA mit 1 mol 2,2,4-Trimethylhexa-methylendiisocyanat ²⁾ Bis-GMA = Additionsprodukt aus Methacrylsäure und Bisphenol-A-diglycidylether ³⁾ Mischung aus Campherchinon (0,2 Gew.-%) und 4-(N,N-Dimethylamino)benzoesäureethylester (2,5 Gew.-%) | | |

Rinderzähne wurden so in Kunststoffzylinder eingebettet, dass sich das Dentin und der Kunststoff in einer Ebene befanden. Nach 15 s Ätzen mit 37 %-iger Phosphorsäure wurde gründlich mit Wasser gespült. Durch die Säureätzung wurden die Dentubuli geöffnet. Dann wurde mit einem kleinen Pinsel (Microbrush) eine Schicht Adhäsiv obiger Zusammensetzung aufgebracht, mit dem Luftbläser zur Entfernung des Lösungsmittels kürz verblasen und für 40 s mit einer Halogenlampe (Astralis® 7, Ivoclar Vivadent AG) belichtet. Auf die Adhäsivschicht wurde ein Zylinder aus einem handelsüblichen Komposit (Tetric® Ceram, Ivoclar Vivadent AG) in zwei Schichten von je 1-2 mm aufpolymerisert.

Anschließend wurden die Prüfkörper 24 h bei 37 °C in Wasser gelagert und die Scherhaftfestigkeit gemäß der ISO-Richtlinie "ISO 1994-ISO TR 11405: Dental materials Guidance on testing of adhesion to tooth structure" bestimmt. Dabei ergab sich für das Adhäsiv mit MAM-EDTA eine Scherhaftfestigkeit von 8,6 MPa, für das Adhäsiv mit MA-EDTA 9,6 MPa. Die Ergebnisse zeigen, dass die erfindungsgemäß verwendeten Alkylendiamin-N,N,N',N'-tetraessigsäure-(meth)acrylamide bei deutlich verbesserter Hydrolysestabilität (vgl. Beispiel 3) eine mit MA-EDTA vergleichbare Scherhaftfestigkeit ergeben.

## Patentansprüche

1. Verwendung eines Alkylendiamin-N,N,N',N'-tetraessigsäure-(meth)acrylamids mit der Formel (I) 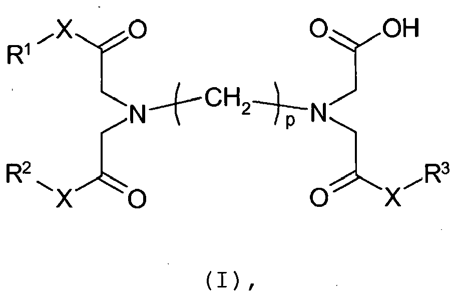 in der
X jeweils unabhängig für O oder NR⁶ steht, R¹, R², R³ unabhängig voneinander für Wasserstoff oder 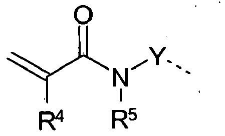 stehen, wobei R¹, R² und R³ nicht gleichzeitig Wasserstoff sind,
Y jeweils unabhängig für einen linearen oder verzweigten C₁- bis C₁₅-Alkylenrest steht, der durch ein oder mehrere O oder S unterbrochen sein kann,
R⁴ jeweils unabhängig für Wasserstoff oder einen C₁- bis C₁₀-Alkylrest steht,
R⁵ jeweils unabhängig für Wasserstoff oder einen C₁- bis C₈-Alkylrest steht,
R⁶ jeweils unabhängig für Wasserstoff oder einen C₁- bis C₆-Alkylrest steht und
p 2 bis 10 ist,
zur Herstellung eines Dentalmaterials.

2. Verwendung nach Anspruch 1, bei der
Y jeweils unabhängig für einen linearen oder verzweigten C₁- bis C₁₀-Alkylenrest steht, der durch ein oder mehrere O oder S unterbrochen sein kann,
R⁴ jeweils unabhängig für Wasserstoff oder einen C₁- bis C₈-Alkylrest steht,
R⁵ jeweils unabhängig für Wasserstoff oder einen C₁- bis C₆-Alkylrest steht,
R⁶ jeweils unabhängig für Wasserstoff oder einen C₁- bis C₄-Alkylrest steht und
p 2 bis 6 ist.

3. Verwendung nach Anspruch 1 oder 2, zur Herstellung eines Adhäsivs oder Zements.

4. Polymerisierbare Zusammensetzung, die
mindestens ein Alkylendiamin-N,N,N',N'-tetraessigsäure-(meth)acrylamid mit der Formel (I) 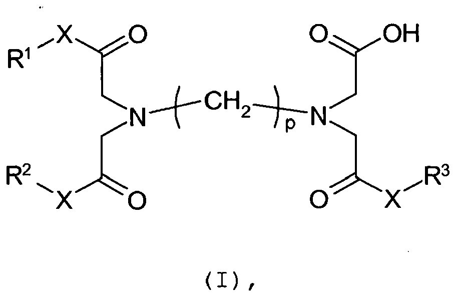 in der
X jeweils unabhängig für O oder NR⁶ steht, R¹, R², R³ unabhängig voneinander für Wasserstoff oder 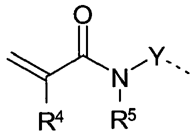 stehen, wobei R¹, R² und R³ nicht gleichzeitig Wasserstoff sind,
Y jeweils unabhängig für einen linearen oder verzweigten C₁- bis C₁₅-Alkylenrest steht, der durch ein oder mehrere O oder S unterbrochen sein kann,
R⁴ jeweils unabhängig für Wasserstoff oder einen C₁- bis C₁₀-Alkylrest steht,
R⁵ jeweils unabhängig für Wasserstoff oder einen C₁- bis C₈-Alkylrest steht,
R⁶ jeweils unabhängig für Wasserstoff oder einen C₁- bis C₆-Alkylrest steht und
p 2 bis 10 ist,
mindestens ein Monomer mit 2 oder mehr polymerisierbaren Gruppen und/oder mindestens ein Monomer mit einer oder mehreren aciden Gruppen und
einen Initiator für die radikalische Polymerisation enthält.

5. Zusammensetzung nach Anspruch 4, bei der
Y jeweils unabhängig für einen linearen oder verzweigten C₁- bis C₁₀-Alkylenrest steht, der durch ein oder mehrere O oder S unterbrochen sein kann,
R⁴ jeweils unabhängig für Wasserstoff oder einen C₁- bis C₈-Alkylrest steht,
R⁵ jeweils unabhängig für Wasserstoff oder einen C₁- bis C₆-Alkylrest steht,
R⁶ jeweils unabhängig für Wasserstoff oder einen C₁- bis C₄-Alkylrest steht und
p 2 bis 6 ist.

6. Zusammensetzung nach Anspruch 4 oder 5, die mindestens ein Monomer mit 2 oder mehr polymerisierbaren Gruppen enthält.

7. Zusammensetzung nach einem der Ansprüche 4 bis 6, die mindestens ein Monomer mit einer oder mehreren aciden Gruppen enthält.

8. Zusammensetzung einem der Ansprüche 4 bis 7, die einen Initiator für die Photopolymerisation enthält.

9. Zusammensetzung nach einem der Ansprüche 4 bis 8, die Füllstoff enthält.

10. Zusammensetzung nach Anspruch 9, wobei der Füllstoff ein partikulärer Füllstoff mit einer mittleren Partikelgröße von 1 nm bis 10 µm ist.

11. Zusammensetzung nach einem der Ansprüche 4 bis 10, die
| | |
|---|---|
| a) 0,5 bis 50 Gew.-% | Alkylendiamin-N,N,N',N'-tetraessigsäure-(meth)acrylamid mit der Formel (I), |
| b) 0,01 bis 15 Gew.-% | Initiator für die radikalische Polymerisation, |
| c) bis zu 80 Gew.-% | weitere polymerisierbare Monomere, |
| d) 0 bis 95 Gew.-% | Lösungsmittel, |
| e) 0 bis 75 Gew.-% | Füllstoff |
enthält.

12. Zusammensetzung nach Anspruch 11, die
| | |
|---|---|
| a) 5 bis 40 Gew.-% | Alkylendiamin-N,N,N',N'-tetraessigsäure-(meth)acrylamid mit der Formel (I), |
| b) 0,1 bis 5 Gew.-% | Initiator für die radikalische Polymerisation, |
| c) bis zu 60 Gew.-% | weitere polymerisierbare Monomere, |
| d) 0 bis 80 Gew.-% | Lösungsmittel, |
| e) 0 bis 60 Gew.-% | Füllstoff enthält. |

13. Zusammensetzung nach Anspruch 12, die monodispersen, nanopartikulären Füllstoff auf Basis von SiO₂, ZrO₂, Al₂O₃, AlO(OH) oder deren Mischoxiden enthält.

14. Zusammensetzung nach Anspruch 12 oder 13, wobei der Füllstoff eine mittlere Partikelgröße von 5 bis 200 nm aufweist.

15. Zusammensetzung nach Anspruch 11, die
| | |
|---|---|
| a) 5 bis 40 Gew.-% | Alkylendiamin-N,N,N',N'-tetraessigsäure-(meth)acrylamid mit der Formel (I), |
| b) 0,1 bis 5 Gew.-% | Initiator für die radikalische Polymerisation, |
| c) bis zu 60 Gew.-% | weitere polymerisierbare Monomere, |
| d) 1 bis 75 Gew.-% | Füllstoff enthält. |

16. Zusammensetzung nach Anspruch 15, die mikrofeinen Füllstoff auf Basis von Quarz-, Keramik-, Glaskeramik- oder Glaspulver enthält.

17. Zusammensetzung nach Anspruch 15 oder 16, wobei der Füllstoff eine mittlere Teilchengröße von 0,01 bis 5 µm aufweist.

18. Zusammensetzung nach einem der Ansprüche 11 bis 17, wobei die Komponente (c) 1 bis 50 Gew.-% Monomer mit einer oder mehreren aciden Gruppen enthält, bezogen auf die Gesamtmasse der Zusammensetzung.

19. Zusammensetzung nach einem der Ansprüche 11 bis 18, wobei die Komponente (c) 1 bis 45 Gew.-% Monomer mit 2 oder mehr polymerisierbaren Gruppen enthält, bezogen auf die Gesamtmasse der Zusammensetzung.

## Claims

1. Use of an alkylenediamine-N,N,N'N'-tetraacetic acid (meth)acrylamide of formula (I) for manufacturing a dental material 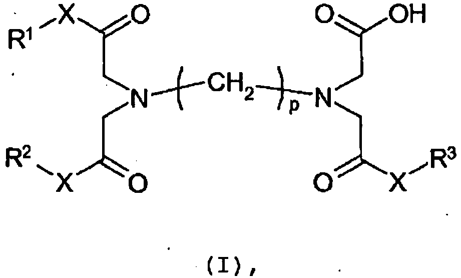 wherein
X in each case independently stands for O or NR⁶, R¹, R², R³ independently of one another stand for hydrogen or 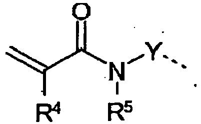 wherein R¹, R² and R³ are not simultaneously hydrogen,
Y in each case independently stands for a linear or branched C₁ to C₁₅ alkylene residue that can be interrupted by one or more O or S,
R⁴ in each case independently stands for hydrogen or a C₁ to C₁₀ alkyl residue, R⁵ in each case independently stands for hydrogen or a C₁ to C₈ alkyl residue, R⁶ in each case independently stands for hydrogen or a C₁ to C₆ alkyl residue and
p is 2 to 10.

2. Use according to claim 1, **characterised in that**
Y in each case independently stands for a linear or branched C₁ to C₁₀ alkylene residue that can be interrupted by one or more O or S,
R⁴ in each case independently stands for hydrogen or a C₁ to C₈ alkyl residue, R⁵ in each case independently stands for hydrogen or a C₁ to C₆ alkyl residue, R⁶ in each case independently stands for hydrogen or a C₁ to C₄ alkyl residue and
p is 2 to 6.

3. Use according to claim 1 or 2, for manufacturing an adhesive or cement.

4. Polymerisable composition according to claim 3, **characterised in that** it comprises
at least one alkylenediamine-N,N,N',N'-tetraacetic acid (meth)acrylamide of the Formula (I) 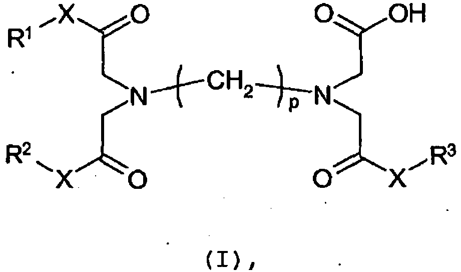 in which
X in each case independently stands for O or NR⁶, R¹, R², R³ independently of one another stand for hydrogen or 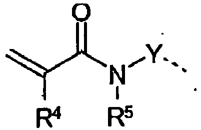 wherein R¹, R² and R³ are not simultaneously hydrogen,
Y in each case independently stands for a linear or branched C₁ to C₁₅ alkylene residue which can be interrupted by one or more O or S,
R⁴ in each case independently stands for hydrogen or a C₁ to C₁₀ alkyl residue,
R⁵ in each case independently stands for hydrogen or a C₁ to C₈ alkyl residue,
R⁶ in each case independently stands for hydrogen or a C₁ to C₆ alkyl residue and
p is 2 to 10,
at least one monomer with 2 or more polymerisable groups and/or at least one monomer with one or more acidic groups and
an initiator for the radical polymerisation.

5. Composition according to claim 4, in which
Y in each case independently stands for a linear or branched C₁ to C₁₀ alkylene residue which can be interrupted by one or more O or S,
R⁴ in each case independently stands for hydrogen or a C₁ to C₈ alkyl residue, R⁵ in each case independently stands for hydrogen or a C₁ to C₆ alkyl residue, R⁶ in each case independently stands for hydrogen or a C₁ to C₄ alkyl residue and
p is 2 to 6.

6. Composition according to claim 4 or 5, **characterised in that** it comprises at least one monomer with 2 or more polymerisable groups.

7. Composition according to one of claims 4 to 6, **characterised in that** it comprises at least one monomer with one or more acidic groups.

8. Composition according to one of claims 4 to 7, **characterised in that** it comprises an initiator for photo-polymerisation.

9. Composition according to one of claims 4 to 8, **characterised in that** it comprises filler.

10. Composition according to claim 8, **characterised in that** the filler is a particulate filler having an average particle size of 1 nm to 10 µm.

11. Composition according to one of claims 4 to 10, **characterised in that** it comprises
| | |
|---|---|
| a) 0.5-50 wt % | alkylenediamine-*N,N,N',N*'-tetraacetic acid (meth)acrylamide according to formula (I), |
| b) 0.01-15 wt % | initiator for radical polymerisation, |
| c) 0-80 wt % | further polymerisable monomers, |
| d) 0-95 wt % | solvent, |
| e) 0-75 wt % | filler. |

12. Composition according to claim 11, **characterised in that** it comprises
| | |
|---|---|
| a) 5 to 40 wt % | alkylenediamine-*N,N,N',N*'-tetraacetic acid (meth)acrylamide according to formula (I), |
| b) 0.1 to 5 wt % | initiator for radical polymerization, |
| c) up to 60 wt % | further polymerizable monomers, |
| d) 0 to 80 wt % | solvent, |
| e) 0 to 60 wt % | filler. |

13. Composition according to claim 12, **characterised in that** it comprises monodisperse, nanoparticulate filler based on SiO₂, ZrO₂, Al₂O₃, AlO(OH) or their mixed oxides.

14. Composition according to claim 12 or 13, **characterised in that** the filler has an average particle size of 5 to 200 nm.

15. Composition according to claim 11, **characterised in that** it comprises
| | |
|---|---|
| a) 5 to 40 wt % | alkylenediamine-N,N,N',N'-tetraacetic acid (meth)acrylamide according to formula (I), |
| b) 0.1 to 5 wt % | initiator for radical polymerisation, |
| c) up to 60 wt % | further polymerisable monomers, |
| d) 1 to 75 wt % | filler. |

16. Composition according to claim 15, **characterised in that** it comprises microfine filler based on powdered quartz, ceramic, glass ceramic or glass powder.

17. Composition according to claim 15 or 16, **characterised in that** the filler has an average particle size of 0.01 to 5 µm.

18. Composition according to one of claims 11 to 17, **characterised in that** component (c) comprises 1 to 50 wt % of monomer having one or more acidic groups, relative to the overall mass of the composition.

19. Composition according to one of claims 11 to 18, **characterised in that** component (c) comprises 1 to 45 wt % of monomer having 2 or more polymerisable groups, relative to the overall mass of the composition.

## Revendications

1. Utilisation d'un (méth)acrylamide d'acide alkylène-diamine-N,N,N',N'-tétraacétique, de formule (I) : 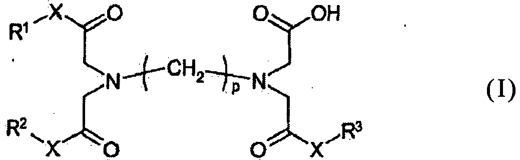 dans laquelle
- X représente, indépendamment en chaque occurrence, un chaînon symbolisé par O ou NR⁶,
- R¹, R² et R³ représentent chacun, indépendamment les uns des autres, un atome d'hydrogène ou un groupe de formule 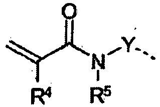 étant entendu que R¹, R² et R³ ne représentent pas tous en même temps un atome d'hydrogène,
- Y représente, indépendamment en chaque occurrence, un groupe alcanediyle en C₁-C₁₅, linéaire ou ramifié, qui peut être interrompu par un ou plusieurs atome(s) d'oxygène ou de soufre,
- R⁴ représente, indépendamment en chaque occurrence, un atome d'hydrogène ou un groupe alkyle en C₁-C₁₀,
- R⁵ représente, indépendamment en chaque occurrence, un atome d'hydrogène ou un groupe alkyle en C₁-C₈,
- R⁶ représente, indépendamment en chaque occurrence, un atome d'hydrogène ou un groupe alkyle en C₁-C₆,
- et l'indice p vaut de 2 à 10,
pour la fabrication d'un matériau dentaire.

2. Utilisation conforme à la revendication 1, pour laquelle
- Y représente, indépendamment en chaque occurrence, un groupe alcanediyle en C₁-C₁₀, linéaire ou ramifié, qui peut être interrompu par un ou plusieurs atome(s) d'oxygène ou de soufre,
- R⁴ représente, indépendamment en chaque occurrence, un atome d'hydrogène ou un groupe alkyle en C₁-C₈,
- R⁵ représente, indépendamment en chaque occurrence, un atome d'hydrogène ou un groupe alkyle en C₁-C₆,
- R⁶ représente, indépendamment en chaque occurrence, un atome d'hydrogène ou un groupe alkyle en C₁-C₄,
- et l'indice p vaut de 2 à 6.

3. Utilisation conforme à la revendication 1 ou 2, pour la fabrication d'un adhésif ou d'un ciment.

4. Composition polymérisable qui contient :
au moins un (méth)acrylamide d'acide alkylène-diamine-N,N,N',N'-tétraacétique, de formule (I) : 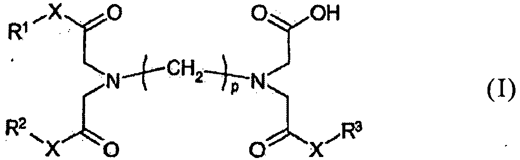 dans laquelle
- X représente, indépendamment en chaque occurrence, un chaînon symbolisé par O ou NR⁶,
- R¹, R² et R³ représentent chacun, indépendamment les uns des autres, un atome d'hydrogène ou un groupe de formule 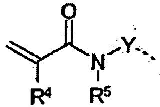 étant entendu que R¹, R² et R³ ne représentent pas tous en même temps un atome d'hydrogène,
- Y représente, indépendamment en chaque occurrence, un groupe alcanediyle en C₁-C₁₅, linéaire ou ramifié, qui peut être interrompu par un ou plusieurs atome(s) d'oxygène ou de soufre,
- R⁴ représente, indépendamment en chaque occurrence, un atome d'hydrogène ou un groupe alkyle en C₁-C₁₀,
- R⁵ représente, indépendamment en chaque occurrence, un atome d'hydrogène ou un groupe alkyle en C₁-C₈,
- R⁶ représente, indépendamment en chaque occurrence, un atome d'hydrogène ou un groupe alkyle en C₁-C₆,
- et l'indice p vaut de 2 à 10,
au moins un monomère doté de deux groupes polymérisables ou plus et/ou au moins un monomère doté d'un ou de plusieurs groupe(s) acide(s),
et un amorceur de polymérisation par voie radicalaire.

5. Composition conforme à la revendication 4, dans laquelle
- Y représente, indépendamment en chaque occurrence, un groupe alcanediyle en C₁-C₁₀, linéaire ou ramifié, qui peut être interrompu par un ou plusieurs atome(s) d'oxygène ou de soufre,
- R⁴ représente, indépendamment en chaque occurrence, un atome d'hydrogène ou un groupe alkyle en C₁-C₈,
- R⁵ représente, indépendamment en chaque occurrence, un atome d'hydrogène ou un groupe alkyle en C₁-C₆,
- R⁶ représente, indépendamment en chaque occurrence, un atome d'hydrogène ou un groupe alkyle en C₁-C₄,
- et l'indice p vaut de 2 à 6.

6. Composition conforme à la revendication 4 ou 5, qui contient au moins un monomère doté de deux groupes polymérisables ou plus.

7. Composition conforme à l'une des revendications 4 à 6, qui contient au moins un monomère doté d'un ou de plusieurs groupe(s) acide(s).

8. Composition conforme à l'une des revendications 4 à 7, qui contient un amorceur de photopolymérisation.

9. Composition conforme à l'une des revendications 4 à 8, qui contient une charge.

10. Composition conforme à la revendication 9, dans laquelle la charge est une charge en particules, et celles-ci présentent une taille moyenne de 1 nm à 10 µm.

11. Composition conforme à l'une des revendications 4 à 10, qui contient :
a) de 0,5 à 50 % en poids de (méth)acrylamide d'acide alkylène-diamine-N,N,N',N'-tétraacétique, de formule (I),
b) de 0,01 à 15 % en poids d'amorceur de polymérisation par voie radicalaire,
c) jusqu'à 80 % en poids d'autres monomères polymérisables,
d) de 0 à 95 % en poids de solvant,
e) et de 0 à 75 % en poids de charge.

12. Composition conforme à la revendication 11, qui contient :
a) de 5 à 40 % en poids de (méth)acrylamide d'acide alkylène-diamine-N,N,N',N'-tétraacétique, de formule (I),
b) de 0,1 à 5 % en poids d'amorceur de polymérisation par voie radicalaire,
c) jusqu'à 60 % en poids d'autres monomères polymérisables,
d) de 0 à 80 % en poids de solvant,
e) et de 0 à 60 % en poids de charge.

13. Composition conforme à la revendication 12, qui contient une charge en nanoparticules, monodispersée, à base de SiO₂, ZrO₂, Al₂O₃, AIO(OH) ou des oxydes mixtes correspondants.

14. Composition conforme à la revendication 12 ou 13, dans laquelle la taille moyenne des particules de charge vaut de 5 à 200 nm.

15. Composition conforme à la revendication 11, qui contient :
a) de 5 à 40 % en poids de (méth)acrylamide d'acide alkylène-diamine-N,N,N',N'-tétraacétique, de formule (I),
b) de 0,1 à 5 % en poids d'amorceur de polymérisation par voie radicalaire,
c) jusqu'à 60 % en poids d'autres monomères polymérisables,
d) et de 1 à 75 % en poids de charge.

16. Composition conforme à la revendication 15, qui contient une charge micro-divisée à base de poudre de quartz, de céramique, de vitrocéramique ou de verre.

17. Composition conforme à la revendication 15 ou 16, dans laquelle la taille moyenne des particules de charge vaut de 0,01 à 5 µm.

18. Composition conforme à l'une des revendications 11 à 17, dans laquelle le composant (c) contient de 1 à 50 %, en poids rapporté au poids total de la composition, d'un monomère doté d'un ou de plusieurs groupe(s) acide(s).

19. Composition conforme à l'une des revendications 11 à 18, dans laquelle le composant (c) contient de 1 à 45 %, en poids rapporté au poids total de la composition, d'un monomère doté de deux groupes polymérisables ou plus.
